# EUROPÄISCHE PATENTSCHRIFT

(11) **EP 3 328 271 B1**
(45) Veröffentlichungstag und Bekanntmachung des Hinweises auf die Patenterteilung: **05.10.2022**
(21) Anmeldenummer: 16757829.3
(22) Anmeldetag: 01.08.2016
(51) Int. Cl.: A61B 5/021

(54) **VERFAHREN ZUR NICHTINVASIVEN BESTIMMUNG VON VITALPARAMETERN EINES LEBENDEN ORGANISMUS**
METHOD FOR NONINVASIVELY DETERMINING VITAL PARAMETERS OF A LIVING ORGANISM
PROCÉDÉ DE DÉTERMINATION NON INVASIVE DE PARAMÈTRES VITAUX D'UN ORGANISME VIVANT

(30) Priorität: 31.07.2015 DE 102015009722; 31.07.2015 DE 102015009721
(43) Veröffentlichungstag der Anmeldung: 06.06.2018
(73) Patentinhaber: Kenkou GmbH, 44799 Bochum (DE)
(72) Erfinder: Redtel, Heiko, 19348 Perleberg (DE)
(74) Vertreter: Schneiders & Behrendt Bochum
(86) Internationale Anmeldenummer: PCT/EP2016/068334
(87) Internationale Veröffentlichungsnummer: WO 2017/021371

(56) Entgegenhaltungen:
- WO-A1-2014/072461
- WO-A1-2015/098977
- US-A1- 2013 218 028
- US-A1- 2014 086 462
- US-A1- 2016 302 735

## Beschreibung

Die Erfindung betrifft ein Verfahren zur nichtinvasiven Bestimmung von Vitalparametern, insbesondere zur einmaligen oder kontinuierlichen Messung und/oder Überwachung des Blutdrucks, eines lebenden Organismus mittels einer Vorrichtung, insbesondere eines Smartgerätes, mit zumindest einer optischen Aufnahmeeinheit und einer Recheneinheit durch Aufnahme einer Abfolge von einzelnen Bilddaten eines beschränkten Bereichs der Haut des lebenden Organismus mittels der optischen Aufnahmeeinheit, Auswertung der Bilddaten, umfassend eine Bestimmung einer Pulswellenlaufzeit und Detektion der Helligkeiten der Pulswelle und Bestimmung von einem oder mehreren Vitalparametern des Organismus aus den Bilddaten mittels der Recheneinheit.

Die WO 2015 / 098977 A1 betrifft Pulswellenmessgeräte mit einer Pulswellenforminformationserfassungseinheit, die Pulswellenforminformation von einem Bereich eines lebenden Körpers optisch erfasst, und einer Pulswellenmerkmalsbetrag-Berechnungseinheit, die einen Pulswellenmerkmalsbetrag basierend auf der Pulswellenforminformation berechnet.

Die US 2013 / 218028 A1 offenbart Systeme und Verfahren zum Bestimmen der arteriellen Pulslaufzeit des Patienten aus einem von diesem Patienten aufgenommenen Quellenvideosignal. In einer Ausführungsform wird ein Videoabbildungssystem verwendet, um ein zeitveränderliches Quellensignal einer proximalen und distalen Region eines Patienten zu erfassen. Die Bilder werden verarbeitet, um lokalisierte Bereiche eines proximalen und distalen Bereichs der Haut des Patienten zu betrachten. Ein Zeitreihensignal für jede der proximalen und distalen Regionen wird aus den Quellvideobildern extrahiert und eine Phase von jedem der extrahierten Signale wird für jede Region berechnet. Eine Phasendifferenz wird zwischen den Zeitreihensignalen der zwei Regionen berechnet, um eine monotone Funktion der Frequenzen in diesen Signalen zu erhalten. Aus der monotonen Funktion wird die arterielle Pulslaufzeit der Person abgeleitet.

Die US 2014 / 086462 A1 betrifft eine Vorrichtung und ein Verfahren zum Verarbeiten von Daten, die von fern detektierter elektromagnetischer Strahlung abgeleitet werden können, die von einem Subjekt emittiert oder reflektiert wird, wobei die Daten physiologische Information umfassen. Insbesondere betrifft US 2014 / 086462 A1 Bildverarbeitungsvorrichtungen und -verfahren zum Erfassen und Überwachen von Vitalparametern in einem interessierenden Objekt.

Eine verwendete Vorrichtung ist insbesondere ein Smartgerät (Smartphone) mit zumindest einer optischen Aufnahmeeinheit und einer Recheneinheit (auch als Datenverarbeitungseinheit zu verzeichnen). Smartphones sind bekanntlich kompakte Computer mit üblicher Architektur bestehend aus CPU, RAN, ROM und Datenbus, mit integrierter Videoaufnahmehardware (Digitalkamera), Bildschirm und Ein-/Ausgabe- und Kommunikationsschnittstellen. Weitere beispielhafte Vorrichtungen sind mit einer Recheneinheit versehene Uhren, Brillen oder andere Bekleidung, auch intelligente Kleidung genannt. Diese Vorrichtung oder Geräte werden meist von einem Anwender bereits für andere Zwecke mitgeführt, beispielsweise um damit zu telefonieren oder im Internet Informationen abzurufen. Somit kann das Verfahren in vorteilhafter Weise mit einer bereits vorhandenen Vorrichtung durchgeführt werden.

Ein Verfahren bzw. eine Vorrichtung der eingangs genannten Art ist beispielsweise aus der WO 2014/072461 bekannt.

Ein Vorteil des dort dargestellten Verfahrens und der Vorrichtung ist darin zu sehen, dass nur ein Messort, und zwar ein einziger beschränkter, zusammenhängender Bereich der Haut des lebenden Organismus (beispielsweise des menschlichen Körpers) notwendig ist. Dabei kann prinzipiell eine beliebige Stelle der Haut verwendet werden. Allerdings ist es vorteilhaft, eine gut durchblutete Stelle zu verwenden, da die Bilddaten so mehr Aussagekraft und ein günstigeres Signal -zu Rausch-Verhältnis aufweisen.

Die Abfolge von einzelnen Bilddaten kann dabei eine Videosequenz sein oder reine Einzelbilder in einer zeitlichen Abfolge. Eine Videosequenz enthält dabei mehr Informationen, die ausgewertet werden können, und verbessert damit das Ergebnis der Bestimmung der Vitalparameter, insbesondere wird die Genauigkeit erhöht. Einzelbilder sind dabei einfacher zu speichern, was insbesondere bei einem begrenzten Speicherplatz beispielsweise in einem Arbeitsspeicher der Recheneinheit von Vorteil ist. Einzelbilder erhöhen darüber hinaus auch die Geschwindigkeit bei der Bestimmung der Vitalparameter, da weniger Information ausgewertet werden muss.

Die Pulswellenlaufzeit wird aus den Bilddaten bestimmt, und zwar indem diese ausgewertet werden. Dies kann beispielsweise durch das Erkennen der durch den Bereich der Haut durchlaufenden Pulswelle und einer damit verbundenen zeitlichen Messung der Pulswellengeschwindigkeit erfolgen. Dabei kann beispielsweise in einem Intervall von einer R-Welle bis zu einer nächsten R-Welle, auch RR-Intervall genannt, gemessen werden. Aus der so bestimmten Pulswellenlaufzeit lassen sich dann die Vitalparameter bestimmen. Die Pulswellenlaufzeit und die Pulswellengeschwindigkeit geben eine Auskunft über die Gefäßsituation. Starre Gefäße mit eingeschränkter Vasomotorik führen zu anderen Laufzeiten und Geschwindigkeiten der Pulswelle. Über die Laufzeit und die Geschwindigkeit der Pulswelle können also Rückschlüsse auf den Zustand der Gefäße getroffen werden. Das ist möglich anhand der Parameter eine arteriosklerotische Veränderung der Gefäße sehr früh zu diagnostizieren und mit der entsprechenden Lebensstiländerung (z.B. fett- und natriumarme Ernährung, sportliche Aktivität) ein Fortschreiten der Arteriosklerose zu verhindern.

Anwendungsbeispiele des vorbekannten Verfahrens sind die Blutdruckmessung in der Überwachung von Personen, die Blutdruckmessung auf der Haut, die Blutdruckmessung und Überwachung im Schlaflabor, die Blutdruckmessung in der Leistungsdiagnostik, die Blutdruckmessung als Dauermessung, beispielsweise über mehrere Stunden oder Tage, Steuerung von Geschwindigkeiten für die Blutabgabe oder die Aufnahme usw.. Der Blutdruck gilt als einer der medizinischen Standards in der Beurteilung der kardiovaskulären Situation in Ruhe und unter körperlicher Belastung. Die physiologischen Grenzwerte in Ruhe und unter Belastung sind umfangreich beschrieben und in Leitlinien festgelegt. Gemäß dem oben genannten Stand der Technik wird der Blutdruck aus der Geschwindigkeit der Pulswelle/der Pulswellenlaufzeit bestimmt. Eine geringe Laufzeit vom Herzen zum Finger steht für einen hohen Blutdruck, da die Gefäße eng gestellt sind. Nach der Eichung der Blutdruckmessung in Ruhe und unter Belastung kann der Blutdruck kontinuierlich gemessen werden. Das vorbekannte Verfahren ist bei allen Personengruppen einsetzbar, durch die nichtinvasive Messung kann jeder ohne Risiko die Messung durchführen. Eine Blutdruckbestimmung über längere Zeit wird nicht nur bei gesunden Sportlern, sondern auch bei Risikogruppen z.B. Herzpatienten und Schwangeren möglich. Die Nutzer erhalten die Möglichkeit, Blutdruckspitzen und Situationen, die zu einem Anstieg führen, zu erkennen. Daraus ergibt sich in der Folge, dass die Nutzer entsprechende Situationen vermeiden können und lernen, ihren Blutdruck durch Änderungen im Lebensstil besser zu kontrollieren.

Aus dem zitierten Stand der Technik ist weiterhin bekannt, die Bestimmung der Pulswellenlaufzeit derart durchzuführen, dass der in den einzelnen Bilddaten wiedergegebene Bereich der Haut in Kacheln unterteilt wird, wonach in jeder der Kacheln der einzelnen Bilddaten eine Farbe, eine Helligkeit und/oder ein Volumen bestimmt wird. Durch Vergleichen der Farbe, Helligkeit und/oder des Volumens der Kacheln in den aufeinanderfolgenden Bilddaten der Abfolge wird ein Änderungsprofil der Farbe, Helligkeit und/oder des Volumens einer jeden Kachel gemäß der Abfolge von Bilddaten bestimmt, wobei das Änderungsprofil eine durch den Bereich der Haut durchlaufende Pulswelle wiedergibt und die Pulswellenlaufzeit aus dem Änderungsprofil berechnet wird.

Darüber hinaus wird in der zitierten Druckschrift beschrieben, dass Bewegungsdaten des menschlichen Körpers mittels zumindest eines Beschleunigungssensors aufgenommen werden. Die Bewegungsdaten erlauben dabei beispielsweise eine Rekonstruktion der körperlichen Belastung der Person, was bei der Auswertung der Bilddaten und bei der Bestimmung der Pulswellenlaufzeit berücksichtigt wird. Die aufgenommenen Bilddaten und Beschleunigungsdaten werden dabei mit einem Zeitstempel versehen und auf einer Speichereinheit der Vorrichtung für eine Langzeitauswertung zusammen mit dem Zeitstempel gespeichert.

Seit Beginn der Messungen von Vitaldaten mit Hilfe von mobilen Endgeräten wie beispielsweise Smartphones wurden so genannte Bewegungsartefakte ständige störende Begleiter innerhalb der gemessenen Zeit des Pulses. Eine Höhenveränderung vom hängenden Arm nach unten nach oben, um beispielsweise auf die Uhr zu schauen, verursacht eine Blutdruckänderung je nach Größe eines Menschen. Bewegungen des Messpunktes beeinflussen signifikant den Blutdruck. Diese durch Bewegungen hervorgerufenen vermeintlichen Störungen im Messverlauf werden herausgeschnitten und nicht vom Datensatz zur Messung beispielsweise von Puls, RR-Intervall, Pulswellenform oder Atmung genutzt. Durch das Verwerfen dieser vermeintlich falschen Daten aus den Bewegungsartefakten entstehen nicht ersetzbare Lücken in den Messdaten, die eine kontinuierliche Messung und Auswertung verhindern. Die Wellendynamik einzelner Pulswellen innerhalb eines RR-Intervalls entsteht durch unterschiedliche Geschwindigkeiten durch die Veränderung des Querschnitts an den Gefäßverzweigungen hin zur Peripherie und durch Bewegungen der Extremitäten.

Wie die Hauptwelle/Systole wird auch die Reflektionswelle/dikrote Welle durch den Weg vom Startpunkt, oder auch dem Reflektionspunkt, wie beispielsweise an der Aortenklappe hin zur Messstelle in die Peripherie "gewaschen" und für den Betrachter als zweidimensionale Helligkeitswelle dargestellt. Alle Pulswellen /RR-Intervalle, die mit Licht und damit aus Gesamthelligkeitswerten am Menschen in Bewegung gemessen werden, unterliegen unterschiedlichsten Gefäßwiderständen und Blutdrucken, beispielsweise auch durch die jeweilige Lage zum so genannten hydrostatischen Indifferenzpunkt (HIP).

Somit werden die Reflektions- und Laufzeiten der Pulswelle/n im Verlauf hin zur peripheren Messstelle beeinträchtigt, bzw. verändert dargestellt und somit falsch interpretiert. Die falsche Interpretation besteht auf der Tatsache, dass die Bewegungen der Extremitäten und die Lage der Messstelle über dem HIP die Helligkeiten drastisch ändern können.

Die Veränderung der Helligkeiten durch Bewegungen wird dabei als Artefakt von den "guten" Ergebnissen getrennt bzw. dem Nutzer nicht angezeigt. Das führt zur Interpolation der entfernten Ergebnisse und damit zu falschen Werten.

Der Erfindung liegt daher die Aufgabe zugrunde, bei der Bestimmung der Vitaldaten und weiterer Messdaten auch diese Artefakte als wertvolle Messwerte in die Auswertung mit einzubeziehen.

Die Erfindung löst diese Aufgabe gemäß dem Merkmal des Patentanspruchs 1 dadurch, dass bei der Pulswellenmessung mit einer bestimmten Lichtfarbe die erfassten Gesamthelligkeiten der Pulswelle mittels geeigneter Farbmodelle in diskrete Farben aufgespalten werden, wobei hierzu die gemessenen Gesamthelligkeiten in das Farbmodell YCbCr umgewandelt und aus diesen Daten in das RGB-Farbmodell umgerechnet werden. Mit dem RGB-Farbmodell wird die Pulswelle dreidimensional dargestellt, wobei den unterschiedlichen Farben zugeordnete Wellen direkt einzeln oder kontinuierlich dem Nutzer angezeigt werden.

Zur Ermittlung einer Pulswelle beispielsweise am Menschen kann das Umgebungslicht, ausgehend von der Sonne, dienen. Um auch ohne und auch im Dunklen die Pulswelle messen zu können, wird zur Ermittlung der Pulswelle künstliches Licht auf die Haut gestrahlt und damit beleuchtet. Dabei wird derzeit künstliches Licht unterschiedlicher Lichtfarbe eingesetzt. Auch nicht sichtbares IR-Licht wird bei der Messung eingesetzt.

Rote Lichtfarbe hat den Vorteil, den größten Anteil von gemessenen Helligkeitsdaten auf der Haut in derzeitigen Messverfahren zu repräsentieren. Da Rot somit den größten Farbraumanteil an den Gesamthelligkeiten repräsentiert, sind die gemessenen Gesamthelligkeiten aus der weißen Lichtfarbe, beispielsweise eines Smartphones, gegenüber der Lichtfarbe Rot fast identisch. Auch grüne und blaue Lichtfarbe wird für die Messung eingesetzt. Blaues Licht hat durch die anstrahlende Energiemenge eine geringere Strahlungstiefe. Auf 1 maximal 2 mm Eindringtiefe in die Haut ist das blaue Licht begrenzt. In diesem Bereich befinden sich häufig Gefäßanteile, die den venösen Bereich repräsentieren. Das gilt insbesondere, wenn der Druck in den unteren Schichten der Haut durch den extremen Blutdruck, der beispielsweise durch eine hängende Hand nach unten erzeugt wird, so über einen relativ längeren Zeitraum gehalten bleibt. Die Messergebnisse mit spezieller blauer Lichtfarbe für eine Pulswellenmessung sind durch den hohen Anteil der Einflussnahme des störenden venösen Kreislaufs am schlechtesten von allen Lichtfarben wie rot, grün und weiß bzw. den jeweiligen Wellenlängen.

Für die Aufnahmen von Smartphone Geräten wird das weiße Kameralicht genutzt, da die Lampe und dessen weißes Licht der Sonnenlichtzusammensetzung am nächsten kommt. Da in den in den Smartphones eingesetzten Kameras Bildsensoren eingesetzt werden, die eine Pixelmatrix aufweisen, der in der Regel eine Bayer-Filtermatrix vorgeschaltet ist, die aus den Farben rot, grün und blau aufgebaut ist, wobei eine derartige Kamera RGB-Kamera genannt wird, ist eine Übertragung der Helligkeiten in Farbmodelle, beispielsweise in das RGB-Modell, wandelbar.

Um eine bekannte Lösung für die Wandlung von gemessenen Helligkeiten in Farbe zu nutzen, soll das YCbCr-Farbmodell Anwendung finden. Dabei steht das YCbCr-Farbmodell für die Aufnahme von Daten und das RGB-Farbmodell steht für die Anzeige der Daten. Die Helligkeiten werden in das YCbCr-Farbmodell gewandelt, wonach aus diesem YCbCr-Farbmodell das RGB-Farbmodell errechnet wird. Mit diesem RGB-Farbmodell wird erfindungsgemäß die Pulswelle dreidimensional dargestellt, wobei den unterschiedlichen Farben zugeordnete Wellen direkt einzeln oder kontinuierlich dem Nutzer angezeigt werden Dies geschieht beispielsweise durch die Möglichkeit einer Ansicht von Pulswellen mehrerer RR-Intervalle, die durch die Aufspaltung der Gesamthelligkeiten in beispielsweise drei Farben, rot, grün und blau mit einem RGB-Farbmodell angezeigt werden.

Wenn nun bei der Pulswellenmessung eine Bewegung des Messortes stattfindet (Bewegung einer Extremität von oben nach unten oder unten nach oben) ergeben sich Veränderungen im Wellenverlauf.

Während bei der Gesamthelligkeit und bei der Farbaufspaltung "rot" ähnliche Verläufe zu sehen sind (beide Pulswellen verlieren durch die Bewegung stark an Helligkeiten), ist bei der Farbe Blau kaum eine Reaktion zu sehen.

Durch die unterschiedlichen Verläufe der "Gesamthelligkeitswelle" und der Farblaufspaltung können die Daten aus den Pulswellen bereinigt und verbessert werden und Bewegungen gemessen werden.

Weiterhin werden die unterschiedlichen Geschwindigkeiten und die Lage des Messortes einer Pulswelle zum HIP deutlich. Denn das Absacken des Blutes in die Extremitäten, beispielsweise in der Hand oder dem Finger, durch das Hineinpressen von Blut durch Beschleunigung hin zum Körper oder auch Fliehkräfte durch Rotation sowie auch durch die Trägheit des Blutes durch die Beschleunigung weg vom Körper erzeugt unterschiedlichste Blutdrücke und damit unterschiedlichste Helligkeiten. Die Vielzahl von durch die Erfindung möglichen Pulswellen innerhalb eines RR-Intervalls lässt die Betrachtung des Gewebes mit dem RGB-Farbmodell aus unterschiedlichen Bereichen/Tiefen des Gewebes zu. Der Abgleich untereinander sowie die Nichtreaktion einer einzelnen Pulswelle gegenüber einer schnellen Bewegung verbessert die kontinuierliche Pulswellenanzeige. Jede einzelne Farbe aus den aufgespaltenen Helligkeiten des Lichts reagiert bei Drücken unterschiedlich auch gegenüber der jeweilig unterschiedlichen Eindringtiefe der Farben an der Messstelle.

Um Ressourcen zu sparen und der Übersichtlichkeit halber können mit Hilfe von Frequenzaufspaltungen einzelne Wellen und Ansichten ausgewählt werden und so für eine günstigere Informationsabgabe innerhalb von Pulswellen eines RR-Intervalls dienen.

Da wie oben ausgeführt die Wellen der farblichen Aufspaltung, die aus der Gesamthelligkeit aus beispielsweise weißer Lichtfarbe aufgespalten und ermittelt werden, sich stark gegensätzlich verhalten, werden die Messfehler der bisherigen zweidimensionalen Ansicht deutlich.

Die Gesamthelligkeitsdaten aus beispielsweise weißer Lichtfarbe, beinhalten mehr als nur eine Qualitätsverbesserung von Daten für eine Pulswellenlaufzeitmessung. Insbesondere lässt sich weit mehr als eine Pulswelle innerhalb eines einzelnen RR-Intervalls oder einer Vielzahl von RR-Intervallen auch als kontinuierliche Messung generieren.

Diese Analytik ist sinnvoll, um Geschwindigkeiten und die jeweiligen Startorte des Pulswellenbeginns festzustellen.

Fehlende oder auf Krankheiten hindeutende Pulswellenamplituden von Pulswellen innerhalb eines RR-Intervalls sowie deren Start- und Laufzeiten, entstanden an Bögen oder arteriellen Verzweigungen, beispielsweise Aortenbogen, oder eine fehlende, geschwächte oder gestörte Windkesselfunktion, spiegeln den Gefäßzustand, die Gefäßaktivität oder auch Wirkungen von Medikamenten wieder. Die bisherigen Messungen in zweidimensionaler Ansicht beinhalten diese Informationen nicht.

Die Zusammensetzungen der RGB-Farben bilden die Summe der Farbe beispielsweise der Haut bzw. Körperfarbe, wobei beispielsweise rot 255, grün 108 und blau 63 für einen gesunden Menschen aus Nordeuropa und bei einer Zimmertemperatur von 21°C, einem Blutdruck 120/80 und einer Herzfrequenz von 80 gilt, wobei der Messpunkt an der Extremität in Höhe des HIP erfolgt.

Um Mischfarben und unterschiedlichste Pulswellen innerhalb eines RR-Intervalls zu ermitteln, können die Anteile von beispielsweise rot 255, grün 108, blau 63 geändert werden. Die gemessenen Lichtfarben, aufgespalten aus den Gesamthelligkeiten, beispielsweise aus weißer Lichtfarbe des Smartphones, werden durch die Farbzusammensetzung beispielsweise rot 252, grün 87, blau 65 ersetzt. Die gesuchte Farbzusammensetzung wird in dem gemessenen Datensatz der Gesamthelligkeiten ermittelt und wiederum als weitere bzw. spezialisierte Welle dargestellt.

Um die Daten effizienter bearbeiten zu können, können die Gesamthelligkeiten wie weiter oben beschrieben gekachelt gemessen und für eine bessere Auswertung gekachelt auch dem Nutzer zur Verfügung gestellt werden.

Da die Startzeiten der reflektierten Pulswellen innerhalb eines RR-Intervalls über die Geschwindigkeit und über die zurückgelegte Strecke durch den Fachmann errechenbar sind, ist es mit der erfindungsgemäßen Methode möglich, beispielsweise fehlende oder krankhafte Pulswellenamplituden von Pulswellen innerhalb eines RR-Intervalls sowie deren Start- und Laufzeiten, zu erkennen und somit den Gefäßzustand, die Gefäßaktivität auch Wirkungen von Medikamenten zu erkennen.

Die Messung stellt somit ein Bindeglied für eine vorzeitige Erkennung von gefährdenden Engpässen im kardiovaskulären System dar. Die erfindungsgemäße Methode bietet jedoch weitere Vorteile.

Um den Blutdruck nicht invasiv kontinuierlich messen zu können, ist die Kenntnis der Laufzeit des Druckausgleiches innerhalb des arteriellen Systems notwendig. Um eine Druckveränderung zu manipulieren, wird die Hand nach unten hängend geführt und wenige Sekunden dort ruhend belassen. Nach wenigen Sekunden wird der Arm nach oben über den Kopf geführt. Die Zeit bis zum Ausgleich des Drucks, sichtbar am Maximum der Helligkeit durch die manipulierte Bewegung, wird gemessen. Die Messung geschieht indem die Gesamthelligkeiten aus dem Smartphone einer Videoaufzeichnung gemessen und gespeichert werden. Gleichzeitig wird mit demselben Zeitstempel eine Bewegungs- und Beschleunigungsmessung durchgeführt und die Ausgleichszeit des Blutdrucks gegenüber dem hydrostatischen Indifferenzpunkt (HIP) ermittelt. Dabei spiegelt der HIP den zentralen Blutdruck wieder. Es ist bekannt, dass der HIP sich unbeeindruckt gegenüber wechselnden Drücke gegenüber der Peripherie verhält. In den Extremitäten ist der Blutdruck je nach Lage, beispielsweise der Hand, verändert. Gemäß der Erfindung wird aus einer Vielzahl von aufgespaltenen Pulswellen und unterschiedlicher Filterung der Daten innerhalb von beispielsweise 1 RR-Intervall das Verhalten von Gefäßen und Gewebe gemessen. Für eine Blutdruckmessung sowie eine Beurteilung des kardiovaskulären Systems muss das kardiovaskuläre System lokal an der Messstelle manipuliert werden. Die Manipulierung geschieht durch die Bewegung von Extremitäten relativ zur Lage zum HIP.

Das Absacken des Blutes in die Extremitäten, beispielsweise der Hand oder dem Finger, das Hineinpressen von Blut durch Beschleunigung hin zum Körper, Fliehkräfte durch Rotation, sowie auch durch die Trägheit des Blutes in den Gefäßen durch die Beschleunigung weg vom Körper erzeugt unterschiedliche aufgespaltene Helligkeiten aus der Gesamthelligkeit.

Die aufgespaltenen Farben aus den Farbmodellen dienen der Beurteilung des Blutdrucks. Jede einzelne Lichtfarbe reagiert bei Drücken unterschiedlich, auch gegenüber der jeweilig unterschiedlichen Eindringtiefe an der Messstelle. Muskulatur oder Fettgewebe sind Träger der Information während der Überleitung des Drucks von den arteriellen Gefäßen hin zum Sitz der Messstelle und somit oberhalb der Haut.

Blaues Licht und auch die aufgespaltene blaue Farbe misst im RGB-Farbmodell die Schichten bis maximal 2mm. In diesem Bereich befinden sich häufig Gefäßanteile, die den venösen Bereich repräsentieren. Unter Umständen kann die Messstelle nur den venösen Puls darstellen. Das gilt insbesondere, wenn der Druck in den unteren Schichten der Haut durch den extremen Blutdruck, beispielsweise erzeugt durch eine hängende Hand nach unten, über einen relativ längeren Zeitraum gehalten bleibt.

Durch die Veränderungen der Lage der Messstelle, beispielsweise auf der Hand eines Menschen in Relation zum HIP entstehen Druckveränderungen in den Gefäßen und im Gewebe und somit auch resultierend eine Farbveränderung der Farben aus dem RGB-Farbmodell. Die Amplitudenhöhen, sowie die Helligkeiten der Farben grün und blau verändern sich gegensätzlich bei Druckveränderungen zu den Gesamthelligkeiten sowie auch zu rot aus dem RGB-Farbmodell. Als separierte Farbanteile, beispielsweise grün, blau und rot lassen sich die Farben zu den jeweiligen Druck- und Druckstandszeiten aufgrund ihrer unterschiedlichen Amplitudenhöhen sehr gut gesondert detektieren. Baut der Blutdruck ab (Hand wird nach oben geführt) beispielsweise in Höhe des HIP bei gemessenem Blutdruck 120/80, springt die Helligkeit des grünen Lichts zusammen mit dem blauen Farbanteil nach oben. Der rote Farbanteil wird dunkler.

Der blaue Farbanteil stellt im Hoch- Normal- und Niederdrucksystem die höchsten messbaren Amplitudenhöhen von RR-Intervallmessungen dar. Die blaue Lichtfarbe reagiert am schnellsten von allen Lichtfarben. Sie ist der Marker für die Ausgleichszeit wechselnder Helligkeiten bei der Blutdruckmessung.

In Ruhe und unter beispielsweise HIP-Bedingungen (120/80) ist grün größtenteils absorbiert und nur sehr gering wahrzunehmen. Der grüne Helligkeitsanteil an den Gesamthelligkeiten mit dem RGB-Farbmodell ermittelt, liegt zwischen 1 bis 3%. Bei sehr hohen Blutdrücken liegt jedoch der Anteil grün weit höher. Ergebnisse mit über 30% sind möglich.

Der rote Anteil und der blaue Anteil werden entsprechend proportional geringer.

Auch die Gesamthelligkeit zeigt einen geringen Amplitudenausschlag, da rot einen Großteil der gespaltenen RGB-Farben bildet (über 70% Anteil).

Die Amplitudenhöhen unterschiedlicher Pulswellen und innerhalb eines RR-Intervalls sind weit höher und auch länger detektierbar als die Gesamthelligkeiten. Wird die Hand mit der Messstelle von oben nach unten oder von unten nach oben geführt, überschneiden sich die grünen und blauen Wellen aufgrund der Reaktionsgeschwindigkeiten auf der Basis zum bestehenden Druck.

Um den Wechsel von Farben und Farbräumen zu beurteilen, wird die Zeit der Zustandsänderung aufgrund der Abhängigkeiten zur Lage zum HIP, der Drücke und der Bewegung mit demselben Zeitstempel gespeichert. Die Zeit bis zur Helligkeitsstabilisierung bzw. bis zum Erreichen der maximalen Helligkeit nach einer Zustandsänderung in Höhe zum HIP hin, wird mit den Messwerten aus den Bewegungen mit Hilfe von Sensoren (Beschleunigungssensoren etc.) abgeglichen. Mit dem Abgleich von Farbwechseln und Farbänderungen der aufgespaltenen Farben gegenüber dem Standort und der Lage der Extremität bzw. der Messstelle können Ergebnisse über Drücke, Gase und Stoffe sowie Geschwindigkeiten definiert werden. Die aufgespaltenen Wellen aus der Gesamthelligkeit einer speziellen Lichtfarbe, z.B. weiß, erklären die Zusammenhänge und Differenzen von Bewegungen und die änderbaren Druckverhältnisse in den Gefäßen und dem Gewebe.

Die Blutdrücke innerhalb der Pulswelle variieren von Schlag zu Schlag in Systole und Diastole, sowohl während des Auswurfs, als auch hin zur Peripherie, beispielsweise zu den Extremitäten wie Hand, Arm oder Finger.

Beispielsweise und bekanntermaßen herrscht im Bereich der reflektierten Pulswelle eines gesunden Menschen ein niedrigerer Blutdruck als im Bereich der Hauptwelle der Systole. Durch die jeweilige Höhe zum HIP verändert sich der Blutdruck in den Extremitäten bzw. der Messstelle und damit die Gesamthelligkeit und damit auch die Farbzusammensetzung der Farbanteile im RGB-Farbmodell. Zum Nachweis und zur Berechnung einer respiratorischen Sinusarythmie RSA kann das RGB-Farbmodell ebenfalls dienen.

Die Helligkeit (weißes Licht) mittels Kamera eines Smartphones gemessen, ermittelt die Pulswellen. Die Atmung ist als große Welle detektierbar, indem eine Vielzahl von Pulswellen gemessen werden. Die absoluten und maximalen Helligkeiten aus den Gesamthelligkeiten der Pulswellen werden erfasst. Ein spezieller Filter detektiert nur die für die Atmungsbeurteilung notwendigen Maxima. Spiegelt die Torusveränderung der steigenden und fallenden Pulse (respiratorische Sinusarhytmie RSA) die Ein- und Ausatmung, so spricht man von einem Bio-Feedback.

Die Atmung kann über die Bauch- oder Brustatmung erfolgen. Die Höhe der Variabilität durch die Atmung ist auch Ausdruck der Anpassungsfähigkeit. Eine mögliche tiefe Atmung spiegelt die Fähigkeit wieder, auch bei größerer Leistungsbeanspruchung eine Anpassung und Ökonomisierung noch vornehmen zu können.

Weißes Licht bzw. die Gesamthelligkeit aus einer zweidimensionalen Messung mit einer roten oder grünen Lichtfarbe gemessen, beispielsweise bei so genannten Wearables, kann die maximale Helligkeitswelle als Atmung interpretieren. Aber spätestens bei der Bewegung von Extremitäten bzw. der Messstelle wird das Erkennen dieser Signale beeinträchtigt. Atemzüge und Druckveränderungen lassen die Messwerte als Artefakte untergehen. Jede einzelne Messung könnte noch interpoliert werden. Die Summe der Fehler macht aber dabei ein Ergebnis unmöglich.

Um die Atmungswelle aus den farblich aufgespaltenen Wellen bei Bewegung messen zu können, bedarf es der grünen Welle. Die grüne Welle spiegelt die tatsächlichen Inhalte des Sauerstoffs zur Füllung bzw. Ladung des Transportmittels Blut in die Peripherie und ist gleichzeitig ein Indikator für hohe Drücke. Eine tiefe Einatmung eines gesunden Menschen und unter normalen Blutdruckverhältnissen zeigt eine kleine Welle mit einem Farbanteil von weniger als 3%. Um die Atmung zu detektieren, ist es nicht notwendig den Puls zu messen. Nur die jeweilig erreichten Gesamthelligkeiten bilden das Ergebnis einer Atmungsfrequenz ab.

Der blaue Anteil detektiert die Restfüllung des Sauerstoffs im Blut über den venösen Kreislauf zurück zum Herzen unter hohen Druckverhältnissen. Die Lage der Messvorrichtung ist zu beachten. Ist noch relativ viel Sauerstoff im Blut, verändert sich die Farbe der blauen Welle aus dem RGB-Farbmodell. Unter hohen Blutdrücken erzielen die Wellen blau und grün einen höheren Gesamtanteil im Farbraum, rot nimmt ab.

Das erfindungsgemäße Verfahren kann aber auch für eine Ermittlung der inhaltlichen Gase, Substanzen und Stoffe an der Messstelle genutzt werden. Beispielsweise werden Reflektionen und Absorptionen für eine Analytik von Zuständen in Gasen, Substanzen oder Stoffen genutzt. Mit bekannten spektrographischen Einheiten und Kenngrößen werden die Farben aus dem RGB-Farbmodell kalibriert. Die genutzten Lampen und deren Lichtstärke werden auf die notwendige Eindringtiefe auf der jeweiligen Messstelle abgestimmt. Somit sind sichtbares und/oder unsichtbares Licht Grundlagen für die Ermittlung von unterschiedlichsten Inhaltstoffen im Gewebe eines lebenden Organismus, beispielsweise eines Menschen, mit Hilfe von gemessenen Helligkeiten aus mobilen Endgerät, beispielsweise einem Smartphone. Der Sauerstoffanteil in den Gefäßen bzw. Geweben kann über den grünen Anteil von Lichtfarbe, auf die spezielle RGB-Farbe kalibriert und anschließend gemessen werden.

Die Erfindung wird im Folgenden anhand von Zeichnungen dargestellt und erläutert.

Es zeigen:
- Fig. 1:: Vier Pulswellen;
- Fig. 2:: Pulswellen gemäß Fig. 1; (1. Interpretation)
- Fig. 3:: Pulswellen gemäß Fig. 1 (2. Interpretation);
- Fig. 4:: zeigt eine andere Darstellung des simulierten Bewegungsausschnitts;
- Fig. 5, Bild 1:: zeigt den simulierten Bewegungsausschnitt in zweidimensionaler Ansicht (Stand der Technik);

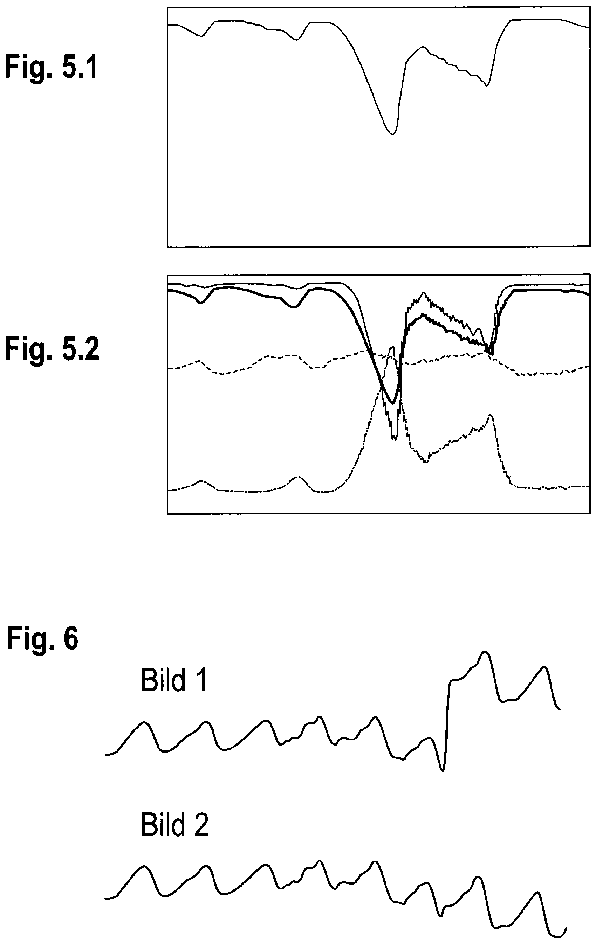

- Fig. 5, Bild 2:: zeigt die dreidimensionale Darstellung nach Farbaufspaltung;
- Fig. 6, Bild 1:: zeigt acht ungefilterte Pulswellen;
- Fig. 6, Bild 2:: zeigt den gefilterten Zustand;

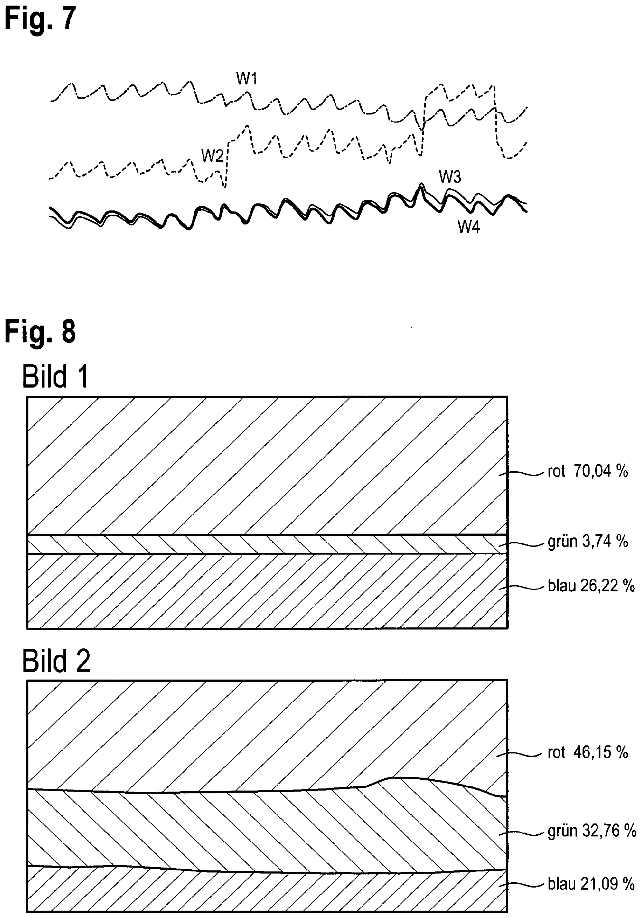

- Fig. 7:: zeigt eine Pulswellenserie mit zwei Gefäß-Druckveränderungen;
- Fig. 8:: zeigt die Beeinflussung des Gewebes bei einem niedrigen Druck (Bild 1) und einem höheren Druck (Bild 2);

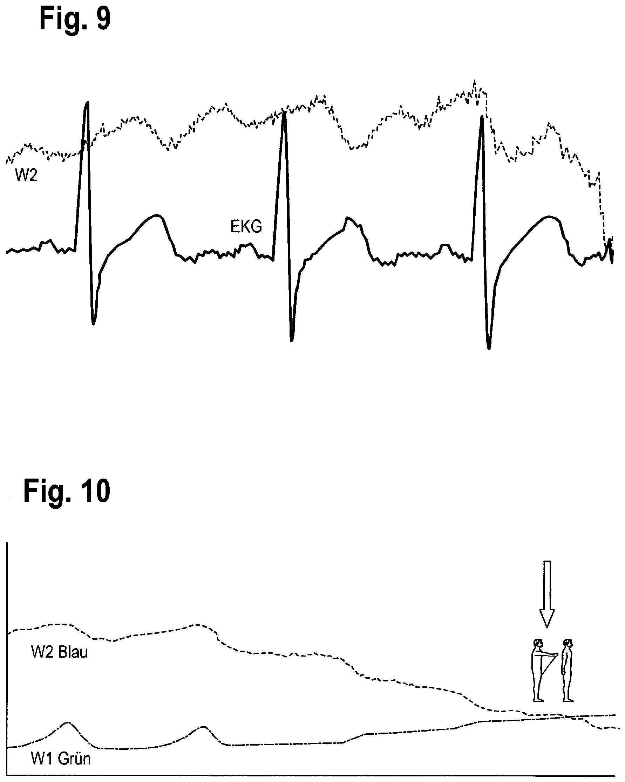

- Fig. 9:: stellt den Zustand dar, wenn der Druck in den unteren Schichten der Haut durch einen extremen Blutdruck über einen relativ langen Zeitraum gehalten bleibt;
- Fig. 10:: Verlauf der blauen und grünen Welle;

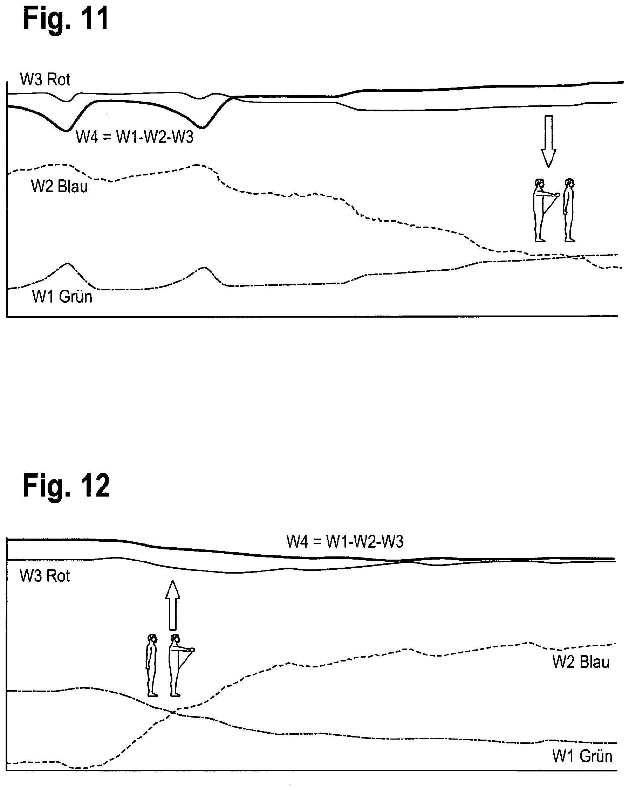

- Fig. 11:: Darstellung wie Fig. 10 mit Rot-Anteil und Gesamthelligkeit (Bewegung nach unten);
- Fig. 12:: Darstellung analog Fig. 11 (Bewegung nach oben).

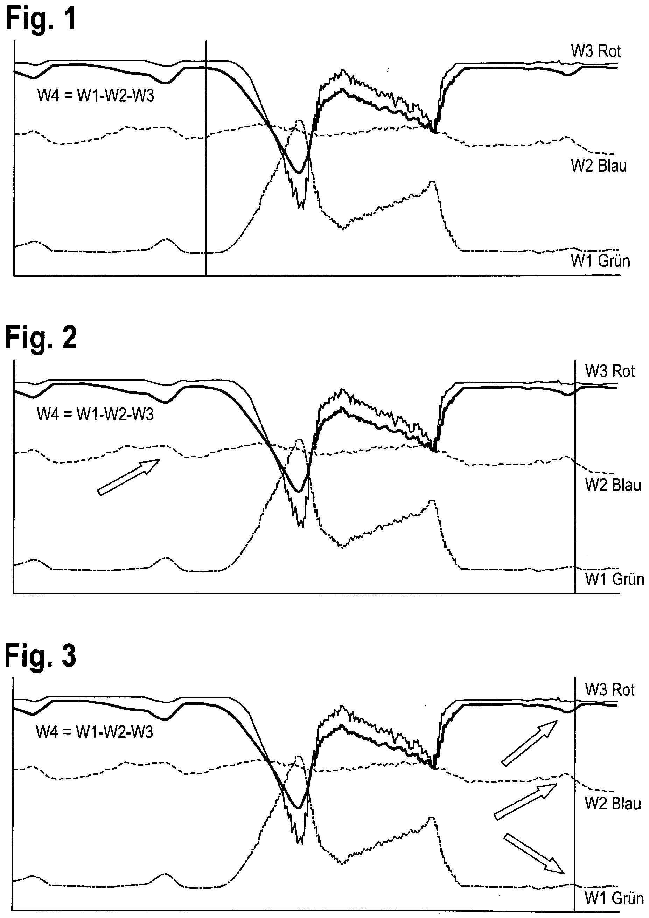

Die Figur 1 zeigt vier Wellen, die aus weißer Lichtfarbe eines Smartphones nach einer Pulswellenmessung in ein RGB-Farbmodell in Farben aufgespalten wurde und in der Mitte der Figur einen deutlichen Ausschlag zeigt. Dieser Ausschlag ist verursacht durch eine simulierte Bewegung des Pulswellenmesspunkts.

W4 ist die Summe aus den Pulswellen W 1, W2 und W3. W4 ist somit die Gesamthelligkeit, während W1 die Farbe grün, W2 die Farbe blau und W3 die Farbe rot repräsentiert.

Wie man aus der Figur entnehmen kann, verhalten sich die Wellen W3 und W4 ähnlich. Beide Pulswellen verlieren durch die Bewegung stark an Helligkeiten. Da die Welle W3 über 70% der Gesamthelligkeit repräsentiert und W1 gegensätzlich an Helligkeit zunimmt, muss die Welle W3 einen höheren Ausschlag in der Amplitude besitzen als die Gesamthelligkeit darstellt.

Wie aus Figur 2 hervorgeht, trägt die Welle W2 mit ähnlich bleibenden Amplituden- Höhen während der Bewegung nur wenig zur Maximierung der Gesamthelligkeit bei. Es ist festzustellen, dass die Welle W4 als Summe für die Gesamthelligkeiten aus einer Pulswellenmessung ermittelt, die tatsächlichen Inhalte der Messung nicht wiedergibt. Die simulierte Bewegung innerhalb einer Pulsmessung würde durch die Gesamthelligkeitsmessung aus der Welle W4 nur als Bewegungsartefakt gedeutet werden können und somit kein RR-Intervall registrieren können.

Auffällig sind auch die unterschiedlichen Startzeiten von Minima zu Maxima einzelner Pulswellenintervalle aus den ermittelten Wellen W1, W2, W3 und der Summe aus allen Wellen W4 wie aus der Figur 3 hervorgeht. W2 und W3 besitzen zeitlich unterschiedliche Peaks. Ebenfalls deutlich in der Figur erkennbar ist, dass die Gesamthelligkeit in der Amplitudenhöhe am geringsten ist. Dieses Ergebnis wäre jedoch bei einer zweidimensionalen Messung als eigentlicher Ausgangswert zu deuten.

Die Figur 3 zeigt mit demselben Zeitstempel versehene Pulswellen /RR-Intervalle W1, W2 und W3 mit unterschiedlichen Startzeiten zur Entstehung einer speziellen Pulswelle, unterschiedliche Gesamthelligkeiten und Amplitudenhöhen und eine sich stetig angepasste prozentuale Verteilung der Farben im RGB-Farbmodell aufgrund einer Bewegung.

Ausgelöst werden die Druckveränderungen auch im peripheren System situationsbedingt, beispielsweise durch das zentrale Nervensystem zur Anpassung an Bewegungen, Hitze, Höhe, Mangelerscheinungen wie Flüssigkeitsverlust, fehlende Energiezufuhr oder Erkrankungen. Die unterschiedlich messbaren Geschwindigkeiten von Pulswellen innerhalb eines RR-Intervalls entstehen beispielsweise durch Druckveränderungen in den Gefäßen selbst und auch als tatsächliche Gefäßwiderstände zur weiteren Übertragung der Drücke auf das Gewebe.

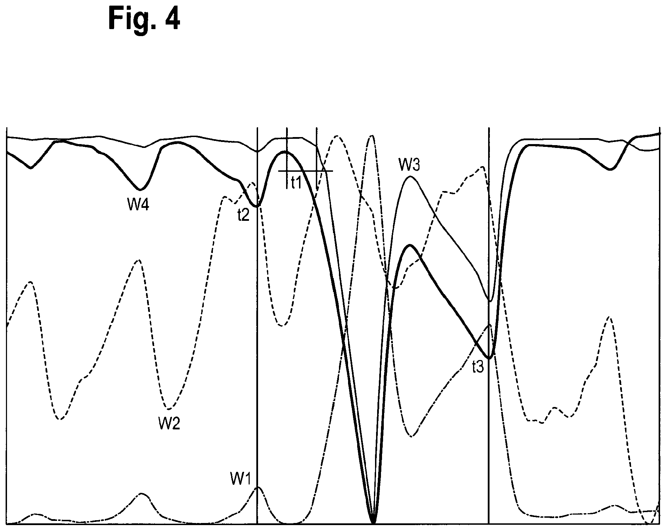

Figur 4 zeigt den gleichen simulierten Bewegungsausschnitt wie in den Figuren 1 bis 3, jedoch in einer anderen Darstellung.

Die Helligkeitswelle W4 aus weißer Lichtfarbe und somit Träger der Gesamthelligkeiten, reagiert auf die Bewegung in einem anderen Verhältnis als W3 aus dem RGB-Modell. Der messbare zeitliche Beginn des Absenkens der Helligkeit ist nicht identisch in beiden Fällen (siehe Figur 4.t1).

Die Welle W2 aus dem RGB-Farbmodell zeichnet die simulierte Bewegung nur schwach mit. Figur 4.t2 zeigt eine andere Startzeit des RR-Intervalls. Deutlich zu erkennen ist in den Gesamthelligkeiten der Welle W4 die Helligkeitsabsenkung inmitten eines RR-Intervalls. Die Pulswelle W2 zeichnet die Pulswelle ohne drastische Helligkeitsverluste oder Helligkeitsgewinne durch. Figur 4.t3 macht die unterschiedlichen Helligkeiten, Pulswellenformen und Zeitabläufe sichtbar. Somit wird aus Figur 4 deutlich, dass in der Gesamthelligkeit weitere Informationen enthalten sind. Bei einer zweidimensionalen "graustufen"-Betrachtung sind die Wellen W1 oder W2 oder W3 aus der Gesamthelligkeitswelle W4 nicht erkennbar.

Figur 5, Bild 1 zeigt aus den Figur 1 bis Figur 4 bekannten simulierten Bewegungsausschnitt nun in zweidimensionaler Ansicht. Die Gesamthelligkeitsanzeige zeigt wie Smartphones, Wearables und Uhren mit heutigen Mitteln nach dem Stand der Technik gemessen.

Die Figur 5, Bild 2 zeigt die unterschiedlichen Wellen aus den RGB-Farben und die sich ständig neu anpassenden Farbenräume an die Situationen und Veränderungen.

In der Figur 6, Bild 1 sind acht ungefilterte Pulswellen dargestellt. Jede einzelne Farbe aus den aufgespaltenen Helligkeiten des Lichts reagiert bei Drücken unterschiedlich auch gegenüber der jeweilig unterschiedlichen Eindringtiefe der Farben an der Messstelle. Somit ist die Druckveränderung Hauptverursacher für Artefakte aus der Bewegung. Um Ressourcen zu sparen, können diese Druckveränderungen oder Bewegungsartefakte so verarbeitet werden, indem spezielle Filter eingesetzt werden. In Figur 6 Bild 1 ist der Gefäßsprung in der Welle W2 erkennbar. Nach der Filterung in Figur 6 Bild 2 wurde der Sprung mit 0,1 Hz gefiltert.

Mit dem Einsatz von Filtern lassen sich Atmungswellen und Gefäßschaltungen separiert messen und je nach Anforderung rechnerisch oder per Bild bzw. Bildserie anzeigen und auswerten.

Die Figur 7 zeigt eine Pulswellenserie mit zwei Gefäß-Druckveränderungen/Sprünge ähnlich wie in Figur 6 Bild 1.

Um Ressourcen zu sparen, können mit Hilfe von Frequenzaufspaltungen einzelne Wellen und Ansichten für eine günstigere Informationsabgabe innerhalb von Pulswellen eines RR-Intervalls dienen.

In Figur 8, Bild 1 und Bild 2 zeigt die Anzeige aus dem RGB-Farbmodell die Beeinflussung des Gewebes gegenüber den Gesamthelligkeiten, sowie die daraus resultierenden aufgespaltenen Helligkeiten des Lichts in RGB-Farben.

Bild 1 zeigt einen niedrigen Druck an. In Figur 8, Bild 2 ist ein hoher Druck festzustellen.

Unter Umständen kann die Messstelle nur den venösen Puls darstellen. Das gilt insbesondere, wenn der Druck in den unteren Schichten der Haut durch den extremen Blutdruck, beispielsweise erzeugt durch eine hängende Hand nach unten, so über einen relativ längeren Zeitraum gehalten bleibt. Dies ist in Figur 9 dargestellt.

In der Figur 10 ist ähnlich wie in Figur 1 dargestellt, dass sich die Farben zu den jeweiligen Druck- und Druckstandzeiten aufgrund ihrer unterschiedlichen Amplitudenhöhen sehr gut gesondert detektieren lassen, hier am Beispiel der Farben grün W1 und blau W2.

Das geht auch aus den Figuren 11 und 12 hervor, wobei in Figur 11 die Bewegung der Extremität von oben nach unten und in Figur 12 diese Bewegung von unten nach oben erfolgt.

## Patentansprüche

1. Verfahren zur nichtinvasiven Bestimmung von Vitalparametern, insbesondere zur einmaligen oder kontinuierlichen Messung und/oder Überwachung des Blutdrucks, eines lebenden Organismus mittels einer Vorrichtung mit zumindest einer optischen Aufnahmeeinheit und einer Recheneinheit, durch Aufnahme einer Abfolge von einzelnen Bilddaten eines beschränkten Bereichs der Haut des lebenden Organismus mittels der optischen Aufnahmeeinheit, Auswertung der Bilddaten mittels der Recheneinheit, umfassend eine Bestimmung einer Pulswellenlaufzeit und Detektion der Helligkeiten der Pulswelle und Bestimmung von einem oder mehreren Vitalparametern des Organismus aus den Bilddaten, wobei bei der Pulswellenmessung mit einer bestimmten Lichtfarbe die erfassten Gesamthelligkeiten der Pulswelle mindestens eines RR-Intervalls mittels geeigneter Farbmodelle in diskrete Farben aufgespalten werden, wobei hierzu die gemessenen Gesamthelligkeiten in das Farbmodell YCbCr umgewandelt und aus diesen Daten in das RGB-Farbmodell umgerechnet werden,
**dadurch gekennzeichnet,**
**dass** mit dem RGB-Farbmodell die Pulswelle dreidimensional dargestellt wird, wobei den unterschiedlichen Farben zugeordnete Wellen direkt einzeln oder kontinuierlich dem Nutzer angezeigt werden.

2. Verfahren nach Anspruch 1, **dadurch gekennzeichnet, dass** eine bewusste Störung durch definierte Bewegung des Messortes mittels Sensorik in die Messung eingeht.

3. Verfahren nach Anspruch 2, **dadurch gekennzeichnet, dass** die Bewegung und/oder Beschleunigung des an einer Extremität angebrachten Messortes in Bezug auf den hydrostatischen Indifferenzpunkt HIP ermittelt wird.

4. Verfahren nach einem der Ansprüche 2 oder 3, **dadurch gekennzeichnet, dass** weitere Sensoren für die Temperaturmessung, die Impedanzmessung und die Zeit verwendet werden.

5. Verfahren nach einem der Ansprüche 2 bis 4, **dadurch gekennzeichnet, dass** die optische Messung und die sensorische Messung mit dem gleichen Zeitstempel versehen sind.

6. Verfahren nach einem der Ansprüche 1 bis 5, **dadurch gekennzeichnet, dass** weißes Licht zur Beleuchtung des Objekts verwendet wird und aus der gemessenen Gesamthelligkeit mit Hilfe der Farbmodelle in die Farben grün, rot und blau aufgespalten wird.

7. Verfahren nach einem der Ansprüche 1 bis 6, **dadurch gekennzeichnet, dass** weißes Licht als gemessene Gesamthelligkeit mit Hilfe der Farbmodelle in eine Vielzahl von Farben aufgespalten wird.

8. Verfahren nach einem der Ansprüche 1 bis 7, **dadurch gekennzeichnet, dass** die Farben aus dem aufgespaltenen Licht für unterschiedliche Pulswellen innerhalb einer Pulswelle /RR-Intervall als Informationsquellen dienen.

9. Verfahren nach einem der Ansprüche 1 bis 8, **dadurch gekennzeichnet, dass** die gemessenen Gesamthelligkeiten aus mindestens einem RR-Intervall mittels einer Kamera als Einzelbild oder Bilderserie mittels des Farbmodells in einzelne Farben aufgespalten werden.

10. Verfahren nach einem der Ansprüche 1 bis 9, **dadurch gekennzeichnet, dass** durch Aufspaltung der gemessenen Gesamthelligkeiten in einzelne Farben mindestens eine farbige Pulswelle ermittelt und deren Geschwindigkeit gemessen wird.

11. Verfahren nach einem der Ansprüche 1 bis 10, **dadurch gekennzeichnet, dass** durch Aufspaltung der gemessenen Gesamthelligkeiten in einzelne Farben, die Farbanteile mit Hilfe von definierten Kennwerten gegenüber einer Spektralskala kalibriert werden.

12. Verfahren nach einem der Ansprüche 1 bis 11, **dadurch gekennzeichnet, dass** die Zeit zur Anpassung und Ökonomisierung des Blutdrucks an und in den Gefäßen und am und im Gewebe an den Extremitäten einen Hinweis auf Druckunterschiede durch die Helligkeitsveränderungen auf und in der Haut gibt.

13. Verfahren nach einem der Ansprüche 2 bis 12, **dadurch gekennzeichnet, dass** die durch die Bewegung des Messpunktes von oben nach unten und umgekehrt verursachte Veränderung der Helligkeit auf und in der Haut Auskunft über die Lage, die Bewegung, die Beschleunigung und die Druckveränderung in den Extremitäten gibt.

14. Verfahren nach einem der Ansprüche 1 bis 13, **dadurch gekennzeichnet, dass** die Pulswellenlaufzeit derart durchgeführt wird, dass der in den einzelnen Bilddaten wiedergegebene Bereich der Haut in Kacheln unterteilt wird, wonach in jeder der Kacheln der einzelnen Bilddaten die Farbe und die Helligkeit bestimmt wird.

15. Verfahren nach einem der Ansprüche 1 bis 14, **dadurch gekennzeichnet, dass** eine Frequenzaufspaltung der Pulswellen durchgeführt wird.

16. Verfahren nach einem der Ansprüche 1 bis 15, **dadurch gekennzeichnet, dass** als Referenzpunkt für die Vitaldatenmessung der hydrostatische Indifferenzpunkt HIP dient.

17. Verfahren nach einem der Ansprüche 1 bis 16, **dadurch gekennzeichnet, dass** aus den Helligkeitsveränderungen an und in den Gefäßen und an und im Gewebe eine Beurteilung des kardiovaskulären Systems erfolgt.

## Claims

1. Method for non-invasive determination of vital parameters of a living organism, in particular for a single or continuous measurement and/or monitoring of blood pressure, by means of a device with at least one optical recording unit and a computing unit, by recording a sequence of individual image data of a limited area of the skin of the living organism by means of the optical recording unit, evaluating the image data by means of the computing unit, comprising determination of a pulse wave propagation time and detection of the brightnesses of the pulse wave and determination of one or more vital parameters of the organism from the image data, wherein in the pulse wave measurement with a certain light colour, the detected total brightnesses of the pulse wave of at least one RR interval are split into discrete colours by means of suitable colour models, wherein the measured total brightnesses are converted for this to the colour model YCbCr and from this data are converted into the RGB colour model,
**characterised in that**
the pulse wave is represented three-dimensionally using the RGB colour model, wherein waves assigned to the different colours are displayed directly singly or continuously to the user.

2. Method according to claim 1, **characterised in that** a deliberate disturbance due to defined movement of the measuring location by means of sensors is included in the measurement.

3. Method according to claim 2, **characterised in that** the movement and/or acceleration of the measuring location applied at an extremity is determined in relation to the hydrostatic indifference point HIP.

4. Method according to one of claims 2 or 3, **characterised in that** other sensors are used for temperature measurement, impedance measurement and the time.

5. Method according to any one of claims 2 to 4, **characterised in that** the optical measurement and the sensor measurement are provided with the same time stamp.

6. Method according to any one of claims 1 to 5, **characterised in that** white light is used for illuminating the object and from the measured total brightness is split with the aid of the colour models into the colours green, red and blue.

7. Method according to any one of claims 1 to 6, **characterised in that** white light as measured total brightness is split with the aid of the colour models into a plurality of colours.

8. Method according to any one of claims 1 to 7, **characterised in that** the colours from the split light for different pulse waves within a pulse wave /RR interval serve as information sources.

9. Method according to any one of claims 1 to 8, **characterised in that** the total brightnesses from at least one RR interval measured by means of a camera as a single image or series of images are split by means of the colour model into individual colours.

10. Method according to any one of claims 1 to 9, **characterised in that** by splitting the measured total brightnesses into individual colours, at least one coloured pulse wave is determined and its velocity measured.

11. Method according to any one of claims 1 to 10, **characterised in that** by splitting the measured total brightnesses into individual colours, the colour portions are calibrated with the aid of defined characteristic values compared with a spectral scale.

12. Method according to any one of claims 1 to 11, **characterised in that** the time for adaptation and rationalisation of the blood pressure on and in the vessels and on and in the tissue at the extremities provides an indication of pressure differences due to the brightness changes on and in the skin.

13. Method according to any one of claims 2 to 12, **characterised in that** the change in brightness on and in the skin caused by the movement of the measuring point from top to bottom and vice versa provides information about the position, the movement, the acceleration and the pressure change in the extremities.

14. Method according to any one of claims 1 to 13, **characterised in that** the pulse wave propagation time is executed in such a way that the area of skin reproduced in the individual image data is divided into tiles, whereupon the colour and the brightness is determined in each of the tiles of the individual image data.

15. Method according to any one of claims 1 to 14, **characterised in that** frequency splitting of the pulse waves is performed.

16. Method according to any one of claims 1 to 15, **characterised in that** the hydrostatic indifference point HIP serves as a reference point for vital data measurement.

17. Method according to any one of claims 1 to 16, **characterised in that** from the changes in brightness on and in the vessels and on and in the tissue, an assessment of the cardiovascular system takes place.

## Revendications

1. Procédé de détermination non invasive de paramètres vitaux, notamment pour la mesure et/ou la surveillance unique ou continue de la pression artérielle, d'un organisme vivant au moyen d'un dispositif comportant au moins une unité d'enregistrement optique et une unité de calcul, par enregistrement d'une succession de données d'image individuelles d'une zone limitée de la peau de l'organisme vivant au moyen de l'unité d'enregistrement optique, évaluation des données d'image au moyen de l'unité de calcul, comprenant une détermination d'un temps de propagation de l'onde de pouls et une détection des luminosités de l'onde de pouls et une détermination d'un ou de plusieurs paramètres vitaux de l'organisme à partir des données d'image ; lors de la mesure de l'onde de pouls avec une couleur de lumière déterminée, les luminosités totales détectées de l'onde de pouls d'au moins un intervalle RR étant décomposées en couleurs discrètes au moyen de modèles de couleurs approprié, les luminosités totales mesurées étant pour cela transformées en modèle de couleurs YCbCr et converties à partir de ces données en modèle de couleurs RVB,
**caractérisé en ce que**
avec le modèle de couleurs RVB, l'onde de pouls est représentée en trois dimensions, les ondes associées aux différentes couleurs étant affichées directement à l'utilisateur, individuellement ou en continu.

2. Procédé selon la revendication 1, **caractérisé en ce qu'**une perturbation volontaire par déplacement défini du lieu de mesure est intégrée dans la mesure au moyen de capteurs.

3. Procédé selon la revendication 2, **caractérisé en ce que** le déplacement et/ou l'accélération du lieu de mesure disposé à une extrémité est déterminé par rapport au point d'indifférence hydrostatique HIP.

4. Procédé selon l'une quelconque des revendications 2 ou 3, **caractérisé en ce que** d'autres capteurs sont utilisés pour la mesure de la température, la mesure de l'impédance et le temps.

5. Procédé selon l'une quelconque des revendications 2 à 4, **caractérisé en ce que** la mesure optique et la mesure par capteurs sont pourvues du même horodatage.

6. Procédé selon l'une quelconque des revendications 1 à 5, **caractérisé en ce que** de la lumière blanche est utilisée pour éclairer l'objet et est décomposée en couleurs verte, rouge et bleue à partir de la luminosité totale mesurée à l'aide des modèles de couleurs.

7. Procédé selon l'une quelconque des revendications 1 à 6, **caractérisé en ce que** de la lumière blanche est décomposée en une pluralité de couleurs en tant que luminosité totale mesurée à l'aide des modèles de couleurs.

8. Procédé selon l'une quelconque des revendications 1 à 7, **caractérisé en ce que** les couleurs de la lumière décomposée servent de sources d'information pour différentes ondes de pouls au sein d'un intervalle d'onde de pouls / RR.

9. Procédé selon l'une quelconque des revendications 1 à 8, **caractérisé en ce que** les luminosités totales mesurées à partir d'au moins un intervalle RR sont décomposées en couleurs individuelles au moyen d'une caméra sous forme d'image unique ou de série d'images au moyen du modèle de couleurs.

10. Procédé selon l'une quelconque des revendications 1 à 9, **caractérisé en ce qu'**au moins une onde de pouls colorée est déterminée par décomposition des luminosités totales mesurées en couleurs individuelles et sa vitesse est mesurée.

11. Procédé selon l'une quelconque des revendications 1 à 10, **caractérisé en ce que** les composantes de couleur sont calibrées à l'aide de valeurs caractéristiques définies par rapport à une échelle spectrale par décomposition des luminosités totales mesurées en couleurs individuelles.

12. Procédé selon l'une quelconque des revendications 1 à 11, **caractérisé en ce que** le temps pour l'adaptation et l'économie de la pression artérielle sur et dans les vaisseaux et sur et dans les tissus aux extrémités donne une indication des différences de pression dues aux variations de luminosité sur et dans la peau.

13. Procédé selon l'une quelconque des revendications 2 à 12, **caractérisé en ce que** la variation de luminosité sur et dans la peau provoquée par le déplacement du point de mesure de haut en bas et inversement donne des renseignements sur la position, le déplacement, l'accélération et la variation de pression dans les extrémités.

14. Procédé selon l'une quelconque des revendications 1 à 13, **caractérisé en ce que** le temps de propagation de l'onde de pouls est effectué de telle sorte que la zone de la peau reproduite dans les données d'image individuelles est divisée en carreaux, après quoi la couleur et la luminosité sont déterminées dans chacun des carreaux des données d'image individuelles.

15. Procédé selon l'une quelconque des revendications 1 à 14, **caractérisé en ce qu'**une décomposition de fréquence des ondes de pouls est effectuée.

16. Procédé selon l'une quelconque des revendications 1 à 15, **caractérisé en ce que** le point d'indifférence hydrostatique HIP sert de point de référence pour la mesure des données vitales.

17. Procédé selon l'une quelconque des revendications 1 à 16, **caractérisé en ce qu'**une évaluation du système cardiovasculaire est effectuée à partir des variations de luminosité sur et dans les vaisseaux et sur et dans les tissus.
